# DEMANDE DE BREVET EUROPEEN

(11) **EP 4 197 600 A1**
(43) Date de publication de la demande: **21.06.2023**
(21) Numéro de dépôt: 22213307.6
(22) Date de dépôt: 14.12.2022
(51) Int. Cl.: A61Q 19/02, A61Q 19/08, A61Q 1/14, A61K 8/92, A61K 8/67, A61K 8/9789, A61K 8/02, A61K 8/25, A61K 8/27, A61K 8/368, A61K 36/73

(54) **KIT DE COMPOSITIONS POUR LE SOIN DE LA PEAU ET SON UTILISATION DANS UNE MÉTHODE DE TRAITEMENT COSMÉTIQUE**

(30) Priorité: 15.12.2021 CH 0707222021
(71) Demandeur: Bioressant Sarl, 1066 Epalinges (CH)
(72) Inventeur: JOHNSTON, Eva, 1162 Saint-Prex (CH); REPOND-GEX, Claudine, 1630 Bulle (CH)
(74) Mandataire: KATZAROV S.A.

(57) **Abrégé**

[La présente invention a trait à la cosmétologie, à la psychodermatologie et à la neurocosmétique. L'invention comprend un kit qui consiste en un ensemble de produits dermo-cosmétiques ayant des bénéfices sur la santé, le confort et l'aspect général de la peau et ayant de plus des bénéfices psychologiques et émotionnels en lien avec l'amélioration de la santé et de l'aspect de la peau. L'invention comprend également un procédé pour améliorer la santé et l'aspect général de la peau d'un sujet et améliorer les aspects psychologiques et émotionnels d'un sujet en lien avec l'amélioration de la santé et de l'aspect de sa peau, ce procédé comprenant une méthode l'application d'un ensemble de produits dermo-cosmétiques appliqués selon des techniques spécifiques sur la peau.

## Description

### Domaine technique

La présente invention a trait à la cosmétologie, à la psychodermatologie et à la neurocosmétique. L'invention comprend un kit qui consiste en un ensemble de produits dermo-cosmétiques ayant des bénéfices sur la santé, le confort et l'aspect général de la peau et ayant de plus des bénéfices psychologiques et émotionnels en lien avec l'amélioration de la santé et de l'aspect de la peau. L'invention comprend également un procédé pour améliorer la santé et l'aspect général de la peau d'un sujet et améliorer les aspects psychologiques et émotionnels d'un sujet en lien avec l'amélioration de la santé et de l'aspect de sa peau, ce procédé comprenant une méthode l'application d'un ensemble de produits dermo-cosmétiques appliqués selon des techniques spécifiques sur la peau.

### Technique antérieure

Les récents développements de la dermatologie ont démontré que la combinaison d'une approche dermatologique et psychologique, connue sous le terme de psycho-dermatologie, apporte des bénéfices supérieurs à ceux obtenus par un traitement des symptômes dermatologiques seuls. Un lien étroit est ainsi démontré entre la peau et les symptômes psychosomatiques. De même, l'on assiste à l'émergence de la neuro-cosmétique, mettant en évidence des liens entre le confort, respectivement l'apparence de la peau d'un individu et ses émotions, ainsi que son bien-être psychologique. La présence de nombreuses terminaisons nerveuses dans la peau sous-tend la relation et l'échange d'information entre le système nerveux et le système cutané.

L'être humain peut avoir jusqu'à 2000 récepteurs par centimètre carré de peau. Les nerfs communiquent avec la peau par l'intermédiaire de transmetteurs neuronaux. Le vieillissement neuronal a un impact sur la communication entre les nerfs et les fibroblastes, mais pas seulement. Dans cette relation complexe entre les nerfs et la peau, un ensemble de métabolismes corporels est impliqué :
GABA, β-endorphines, kératinocytes, mélanocytes, etc.

Il existe un passage d'information clair entre le système cutané et le système nerveux. Ainsi des solutions pour le traitement de la peau en se focalisant sur les émotions semble être l'une des prochaines évolutions de la cosmétique.

De nombreux soins sont disponibles commercialement et démontrent des bénéfices en matière de confort et de rajeunissement de la peau. Ainsi il existe de nombreux produits contre les rides, les rougeurs, les irritations les démangeaisons et les taches foncées (lentigines solaires, taches de rousseur et taches de vieillissement (taches brunes)).

Parmi les ingrédients couramment utilisés dans de tels produits, on peut notamment citer la vitamine A et de la vitamine E.

Il existe toutefois un besoin de développer des soins cosmétiques permettant d'améliorer le confort et l'apparence de la peau de manière holistique, tout en générant une amélioration des émotions du sujet. Il existe un besoin de développer des combinaisons spécifiques de soins cosmétique et d'élaborer une séquence et une technique d'application permettant d'optimiser les effets des ingrédients individuels utilisés.

Il existe également un besoin d'améliorer la naturalité et la sécurité de tels soins cosmétiques, en réduisant l'usage d'agents conservateurs

### Résumé de l'invention

L'objet de l'invention est un soin cosmétique ayant démontré son efficacité en vue de raffermir et d'améliorer l'apparence de la peau, tout en favorisant les ingrédients naturels, tels que les huiles et les infusions de plantes et de fleurs, et nécessitant un minimum d'agents conservateur.

D'une part, l'invention porte sur un kit de traitement cosmétique de la peau comprenant au moins :
a) Un lait démaquillant et nourrissant ayant un pH de 5,3 et comprenant :
   i. de l'eau;
   ii. des triglycérides saturés à chaîne moyenne;
   iii. du monostéarate de glycérol;
   iv. du propylène glycol;
   v. de l'alcool cétylique;
   vi. du diméticone;
   vii. de l'huile essentielle de mandarine verte;
   viii. de l'acétate d'α-tocophérol (vitamine E);
   ix. du carbopol;
   x. de l'EDTA;
   xi. du sorbate de potassium; et
   xii. de l'hydroxyde de sodium;
b) Une composition pour le traitement des capillaires comprenant
   i. une huile végétale de *Rosa moschata;*
   ii. une huile de *Cupressus sempervirens;*
   iii. une huile de fleur de *Chamaemelum nobile;*
   iv. une huile de fleur d'*Helichrysum italicum;*
   v. une huile de Géranium bourbon; et
   vi. de l'acétate d'α-tocophérol (vitamine E);
c) Un peeling pour le visage ayant un pH de 2,7 à 2,8 et comprenant
   i. du monostéarate de glycérol;
   ii. de l'alcool cétylique;
   iii. des triglycérides saturés à chaines moyennes;
   iv. une composition comprenant de l'alcool cétéarylique, du sodium cétéaryl sulfate;
   v. du propylène glycol;
   vi. de l'eau;
   vii. du sorbate de potassium;
   viii. du xanthane,
   ix. de l'acide salicylique;
   x. un agent exfoliant contenant du polyéthylène et du stéarate de zinc;
   xi. une huile essentielle de *Cymbopogon martini;* et
   xii. une huile essentielle de menthe;
d) Un masque dont le pH est de 6,3 et comprenant
   i. du monostéarate de glycérol;
   ii. de l'alcool cétylique;
   iii. des triglycérides à chaînes moyennes;
   iv. une composition comprenant de l'alcool cétéarylique, du sodium cétéaryl sulfate;
   v. une composition comprenant de l'huile de graines de *Ricinus Communis,* de l'huile de ricin hydrogénée et de la cire de *Copernicia Cerifera;*
   vi. de l'extrait de concombre;
   vii. du beurre de karité;
   viii. du propylène glycol;
   ix. de l'eau distillée;
   x. du sorbate de potassium;
   xi. un l'extrait liquide *d'Alchemilla vulgaris;*
   xii. une l'huile essentielle de *Citrus reticulata;* et
   xiii. de l'acétate d'α-tocophérol (vitamine E);
e) Un masque à l'argile consistant en une pâte comprenant de l'eau mélangée à une argile verte ayant un contenu en Silicium compris entre 60 et 75% en poids et comprenant également du fer, du calcium, du manganèse, de l'azote et du potassium;
f) Une crème comprenant
   i. du monostéarate de glycérol;
   ii. de l'alcool cétylique;
   iii. des triglycérides saturés à chaînes moyennes;
   iv. une composition comprenant de l'alcool cétéarylique, du sodium cétéaryl sulfate;
   v. une composition comprenant de l'huile de graines de *Ricinus Communis,* de l'huile de ricin hydrogénée et de la cire de *Copernicia Cerifera;*
   vi. du palmitate cétylique;
   vii. du glycérol;
   viii. du propylène glycol;
   ix. de l'eau distillée;
   x. du sorbate de potassium;
   xi. un extrait liquide *d'Alchemilla vulgaris;*
   xii. de l'alcool benzylique
   xiii. une huile essentielle de *Citrus reticulata;*
   xiv. de l'acétate d'α-tocophérol (vitamine E); et
   xv. de l'oxyde de zinc.

D'autre part, l'invention porte sur un procédé pour le raffermissement de la peau, pour l'amélioration de l'apparence de la peau, pour l'amélioration de l'hydratation de la peau, pour la réduction des lentigines solaires, taches de rousseur et taches de vieillissement (taches brunes), pour la réduction de la ptose de la peau, pour la réduction des rides, pour le remplissage des sillons naso-géniens, pour l'amélioration des émotions, pour l'amélioration de l'équilibre psycho-émotionnel, pour la réduction de l'anxiété et/ou pour la réduction de l'irritabilité d'un individu comprenant
a) l'application sur la peau d'un lait démaquillant et nourrissant ayant un pH de 5,3 et comprenant:
   i. de l'eau;
   ii. des triglycérides saturés à chaîne moyenne;
   iii. du monostéarate de glycérol;
   iv. du propylène glycol;
   v. de l'alcool cétylique;
   vi. du diméticone;
   vii. de l'huile essentielle de mandarine verte;
   viii. de l'acétate d'α-tocophérol (vitamine E);
   ix. du carbopol;
   x. de l'EDTA;
   xi. du sorbate de potassium; et
   xii. de l'hydroxyde de sodium; puis
b) l'application sur la peau d'un peeling pour le visage ayant un pH de 2,7 à 2,8 et comprenant
   i. du monostéarate de glycérol;
   ii. de l'alcool cétylique;
   iii. des triglycérides saturés à chaines moyennes;
   iv. une composition comprenant de l'alcool cétéarylique, du lauryl sulfate de sodium et du sulfate de cétéaryl;
   v. du propylène glycol;
   vi. de l'eau;
   vii. du sorbate de potassium;
   viii. du xanthane,
   ix. de l'acide salicylique;
   x. un agent exfoliant contenant du polyéthylène et du stéarate de zinc;
   xi. une huile essentielle de *Cymbopogon martini;* et
   xii. une huile essentielle de menthe; puis
c) le rinçage complet du peeling appliqué dans l'étape c), de manière à ne laisser aucune trace dudit peeling sur la peau;
d) l'application sur la peau d'une composition pour le traitement des capillaires comprenant
   i. une huile végétale de *Rosa moschata;*
   ii. une huile de *Cupressus sempervirens;*
   iii. une huile de fleur de *Chamaemelum nobile;*
   iv. une huile de fleur d'*Helichrysum italicum;*
   v. une huile de Géranium bourbon; et
   vi. de l'acétate d'α-tocophérol (vitamine E); puis
e) l'application sur la peau durant 20 à 30 minutes d'un masque dont le pH est de 6,3 et comprenant
   i. du monostéarate de glycérol;
   ii. de l'alcool cétylique;
   iii. des triglycérides à chaînes moyennes;
   iv. une composition comprenant de l'alcool cétéarylique, du sodium cétéaryl sulfate;
   v. une composition comprenant de l'huile de graines de *Ricinus Communis,* de l'huile de ricin hydrogénée et de la cire de *Copernicia Cerifera;*
   vi. de l'extrait de concombre;
   vii. du beurre de karité;
   viii. du propylène glycol;
   ix. de l'eau distillée;
   x. du sorbate de potassium;
   xi. un l'extrait liquide *d'Alchemilla vulgaris;*
   xii. une l'huile essentielle de *Citrus reticulata;* et
   xiii. de l'acétate d'α-tocophérol (vitamine E); puis
f) le rinçage à l'eau tiède du masque appliqué dans l'étape e);
g) l'application sur la peau, à l'exception des yeux, des paupières et des lèvres, pendant 15 à 25 minutes, d'un masque à l'argile consistant en une pâte comprenant de l'eau mélangée à une argile verte ayant un contenu en Silicium compris entre 60 et 75% en poids et comprenant également du fer, du calcium, du manganèse, de l'azote et du potassium;
h) le retrait du masque à l'argile, puis
i) l'application sur la peau d'une crème comprenant
   i. du monostéarate de glycérol;
   ii. de l'alcool cétylique;
   iii. des triglycérides saturés à chaînes moyennes;
   iv. une composition comprenant de l'alcool cétéarylique, du sodium cétéaryl sulfate;
   v. une composition comprenant de l'huile de graines de *Ricinus Communis,* de l'huile de ricin hydrogénée et de la cire de *Copernicia Cerifera;*
   vi. du palmitate cétylique;
   vii. du glycérol;
   viii. du propylène glycol;
   ix. de l'eau distillée;
   x. du sorbate de potassium;
   xi. un extrait liquide *d'Alchemilla vulgaris;*
   xii. de l'alcool benzylique;
   xiii. une huile essentielle de *Citrus reticulata;*
   xiv. de l'acétate d'α-tocophérol (vitamine E); et
   xv. de l'oxyde de zinc.

### Description détaillée de l'invention

En premier lieu, l'invention porte sur un kit de traitement cosmétique de la peau comprenant :
a) un lait démaquillant et nourrissant;
b) une composition pour le traitement des capillaires;
c) un peeling;
d) un masque à la vitamine E;
e) un masque à l'argile;
f) une crème;
g) de manière optionnelle une lotion énergisante; et
h) de manière optionnelle un sérum régénérant.

Le lait démaquillant et nourrissant a un pH de 5,3 et comprend :
i. de l'eau, de préférence à une concentration de 66 à 85% en poids, de préférence à une concentration de 80% en poids;
ii. des triglycérides saturés à chaîne moyenne, de préférence à une concentration de 8 à 15% en poids, de préférence à une concentration de 8,4% en poids;
iii. du monostéarate de glycérol, de préférence à une concentration de 6 à 10% en poids, de préférence à une concentration de 7% en poids;
iv. du propylène glycol, de préférence à une concentration de 1 à 1,5 % en poids, de préférence à une concentration de 1,25% en poids;
v. de l'alcool cétylique, de préférence à une concentration de 1,2 à 1,8% en poids, de préférence à une concentration de 1,4% en poids;
vi. du diméticone, de préférence à une concentration de 0,1 à 0,5% en poids, de préférence à une concentration de 0,3% en poids;
vii. de l'huile essentielle de mandarine verte, de préférence à une concentration de 0,15 à 0,4% en poids, de préférence à une concentration de 0,25 en poids;
viii. de l'acétate d'α-tocophérol (vitamine E), de préférence à une concentration de 0,02 à 0.08% en poids, de préférence à une concentration de 0,05% en poids;
ix. du carbopol, de préférence à une concentration de 0,15 à 0,45% en poids, de préférence à une concentration de 0,3% en poids;
x. du sorbate de potassium, de préférence à une concentration de 0,02 à 0,1% en poids, de préférence à une concentration de 0,03 en poids;
xi. de l'EDTA, de préférence à une concentration de 0,05 à 0,1% en poids, de préférence à une concentration de 0,06% en poids; et
xii. de l'hydroxyde de sodium, de préférence à une concentration de 0,5 à 1,3% en poids, de préférence à une concentration de 1 % en poids.

Ce produit a un effet adoucissant sur la peau et la prépare aux soins ultérieurs. La vitamine E nourrit la peau et l'huile d'écorce de mandarine est importante pour permettre au sujet traité de se détendre. La relaxation du sujet est l'une des clés de la réussite du soin. En son absence, les hormones du stress empêcheraient une bonne absorption des principes actifs et la peau resterait "fermée".

La seconde composition présente dans le kit de la présente invention est une composition pour le traitement des capillaires comprenant :
i. une huile végétale de *Rosa moschata* (rose musquée), de préférence à une concentration de 78 à 92,8 % en poids, de préférence à une concentration de 87 % en poids;
ii. une huile de *Cupressus sempervirens* (cyprès), de préférence à une concentration de 4 à 12 % en poids, de préférence à une concentration de 7,74 % en poids;
iii. une huile de fleur de *Chamaemelum nobile* (camomille romaine), de préférence à une concentration de 2,5 à 7 % en poids, de préférence à une concentration de 4,05 % en poids;
iv. une huile de fleur d'*Helichrysum italicum* (helichryse italienne), de préférence à une concentration de 0,4 à 1,2 % en poids, de préférence à une concentration de 0,6 % en poids;
v. une huile de Géranium bourbon, de préférence à une concentration de 0,3 à 1 % en poids, de préférence à une concentration de 0,6 % en poids; et
vi. de l'acétate d'α-tocophérol (vitamine E), de préférence à une concentration de 0,004 à 0,02, de préférence à une concentration de 0,01 % en poids.

La base de cette composition pour les capillaires est la rose musquée (*Rosa moschata).* La composition contient aussi du géranium, notamment Géranium bourbon.

L'ingrédient clé est l'huile de *Rosa moscata* (rose musquée), qui est connue pour ses propriétés cicatrisantes, et donc améliorant la texture de la peau, ainsi que ses propriété antioxydantes. L'huile de *Rosa moscata* est riche en acide transrétinoïque, en cétones, en vitamines A, C, B1 et B2 et en acides gras polyinsaturés Oméga 6 et Oméga 3, accompagnée d'une forte concentration d'antioxydants naturels, comme la vitamine E et le β-carotène. La vitamine E et la vitamine A jouent un rôle important dans le rajeunissement de la peau. Il existe deux types de vitamine A : les rétinoïdes (vitamine A préformée) et les caroténoïdes (vitamine A proformée). La peau est un organe sensible aux rétinoïdes, capable d'absorber facilement la vitamine A lorsqu'elle est appliquée localement. Le rétinol stimule la production de nouvelles cellules cutanées. De nombreuses études cliniques ont confirmé que le rétinol est efficace pour stimuler la production de collagène et réduire les rides lorsqu'il est utilisé par voie topique. Il contribue à unifier le teint de la peau en estompant les taches foncées telles que les lentigines solaires, taches de rousseur et taches de vieillissement (taches brunes). Les acides oléique, linoléique et linolénique sont essentiels pour une peau saine, éclatante et bien hydratée.

La vitamine E est une vitamine liposoluble qui remplit divers rôles dans l'organisme. En tant que vitamine antioxydante, elle aide à neutraliser les espèces réactives de l'oxygène (ERO) qui pourraient avoir des effets néfastes. La vitamine E contribue également à faciliter la fonction immunitaire, et des recherches suggèrent qu'elle pourrait être impliquée dans une variété d'autres réactions biochimiques liées au métabolisme. La vitamine E est une vitamine essentielle, ce qui signifie qu'elle ne peut être synthétisée par l'organisme et qu'elle doit être consommée sous forme d'aliments ou de suppléments. Toutefois, en tant que vitamine liposoluble, la vitamine E peut être stockée dans l'organisme. Chez l'homme, une partie est stockée dans la peau où elle joue un rôle important d'antioxydant. L'utilisation topique de la vitamine E procure également des bénéfices en matière de photoprotection, en réduisant l'impact des rayons UV nocifs sur la peau.

L'immortelle (*Helichrysum italicum*) contribue au recyclage cellulaire et favorise ainsi une régénération saine des cellules de la peau, stimule la production de collagène, réduit les oedèmes, améliore la circulation sanguine, réduit l'inflammation et aide à la cicatrisation. L'immortelle est également riche en acétate de néryle (présent dans l'huile essentielle), et possède ainsi des propriétés antalgiques.

Parmi les autres ingrédients actifs présents dans cette composition, on compte L'italidione, qui joue un rôle dans le traitement des hématomes, les monoterpènes et sesquiterpènes, les différents types de pinène, l'eucalyptol, le menthol naturel, le B-farbesebe, les terpinéols, qui possèdent des propriétés anti-inflammatoires, et le carvéol. L'α-pinène et le β-pinène sont des substances antimicrobiennes et anti-inflammatoires et stimulant la mémoire. L'α-pinène possède également une activité inhibitrice de l'acétylcholinestérase, qui favorise la mémoire avec une CI50 observée de 0,44 mM.

L'eucalyptol, qui est également présent dans cette composition, a des propriétés anti-inflammatoires et antioxydantes.

Le *Cupressus sempervirens* produit un arôme pur qui a un effet à la fois vivifiant et ancrant sur les émotions. Les principaux constituants sont l'α-pinène (68,0 %), l'épicédrol (6,1 %), l'acétate d'α-terpényle (3,5 %) et le germacrène D (2,5 %). Ces huiles essentielles sont également actives pour réduire la dépression, l'anxiété et la fatigue.

La composition pour le traitement des capillaires a un effet calmant et hydratant prononcé.

Le kit de la présente invention comprend également un peeling pour le visage ayant un pH de 2,7 à 2,8 et comprenant :
i. du monostéarate de glycérol, de préférence à une concentration de 2 à 8 % en poids, de préférence à une concentration de 6,4 % en poids;
ii. de l'alcool cétylique de préférence à une concentration de 3,5 à 7 % en poids, de préférence à une concentration de 5,66 % en poids;
iii. des triglycérides saturés à chaines moyennes, de préférence à une concentration de 4 à 8 % en poids, de préférence à une concentration de 6,02 % en poids;
iv. une composition comprenant de l'alcool cétéarylique, du sodium cétéaryl sulfate, de préférence à une concentration totale de 1,5 à 4,5 % en poids, de préférence à une concentration totale de 3 % en poids;
v. du propylène glycol, de préférence à une concentration de 5 à 12,5 % en poids, de préférence à une concentration de 10 % en poids;
vi. de l'eau, de préférence à une concentration de 50 à 80 % en poids, de préférence à une concentration de 61,5 % en poids;
vii. du sorbate de potassium, de préférence à une concentration de 0,07 à 0,5 % en poids, de préférence à une concentration de 0,25 % en poids;
viii. du xanthane, de préférence à une concentration de 0,05 à 0,35 % en poids, de préférence à une concentration de 0,17 % en poids;
ix. de l'acide salicylique, de préférence à une concentration de 0,5 à 4 % en poids, de préférence à une concentration de 3,6 % en poids;
x. un agent exfoliant contenant du polyéthylène et du stéarate de zinc, de préférence à une concentration de 1 à 6 % en poids, de préférence à une concentration de 3 % en poids ;
xi. une huile essentielle de *Cymbopogon martini,* de préférence à une concentration de 0,02 à 0,4 % en poids, de préférence à une concentration de 0,2 % en poids; et
xii. une huile essentielle de menthe, de préférence à une concentration de 0,05 à 0,3 % en poids, de préférence à une concentration de 0,2 % en poids.

Les composants clés de ce peeling sont l'acide salicylique, l'huile de menthe et l'huile de palmarose (*Cymbopogon martini*)*.* Cette combinaison unique exfolie en profondeur la peau et exerce en même temps une action apaisante, hydratante et cicatrisante. Les traumatismes mécaniques ou chimiques à la peau sont évités et l'aspect de la peau est visiblement amélioré. Afin d'optimiser les bénéfices de ce peeling, l'agent exfoliant se compose de préférences de microparticules ayant une taille maximale de 350 µm.

La menthe possède des propriétés favorables à la relaxation et agit comme modulateur d'humeur.

La palmarose est efficace pour le traitement des infections bactériennes, des mycoses, de l'eczéma et d'autres troubles cutanés. Dans le présent soin cosmétique, elle minimise les effets secondaires lors de l'application du produit et réduit notamment la fatigue et les irritations.

Ce peeling se caractérise pour la présence d'ingrédients exclusivement naturels.

Le kit de la présente invention comprend également un masque dont le pH est de 6,3 et comprenant :
i. du monostéarate de glycérol, de préférence à une concentration de 2 à 6% en poids, de préférence à une concentration de 4% en poids;
ii. de l'alcool cétylique, de préférence à une concentration de 3 à 8% en poids, de préférence à une concentration de 6% en poids;
iii. des triglycérides à chaînes moyennes, de préférence à une concentration de 4 à 9% en poids, de préférence à une concentration de 7,5% en poids;
iv. une composition comprenant de l'alcool cétéarylique, du sodium cétéaryl sulfate, de préférence à une concentration de 2,5 à 6,5% en poids, de préférence à une concentration de 4% en poids;
v. une composition comprenant de l'huile de graines de *Ricinus Communis,* de l'huile de ricin hydrogénée et de la cire de *Copernicia Cerifera,* de préférence à une concentration de 10 à 25% en poids, de préférence à une concentration de 15% en poids;
vi. de l'extrait de concombre, de préférence à une concentration de 5 à 10% en poids, de préférence à une concentration de 8% en poids;
vii. du beurre de karité, de préférence à une concentration de 1,5 à 4% en poids, de préférence à une concentration de 2,5% en poids;
viii. du propylène glycol, de préférence à une concentration de 2,5 à 7,5% en poids, de préférence à une concentration de 5% en poids;
ix. de l'eau distillée, de préférence à une concentration de 20 à 64% en poids, de préférence à une concentration de 34.65% en poids;
x. du sorbate de potassium, de préférence à une concentration de 0,05 à 0,3% en poids, de préférence à une concentration de 0,15% en poids;
xi. un l'extrait liquide d'*Alchemilla vulgaris,* de préférence à une concentration de 5 à 10% en poids, de préférence à une concentration de 8% en poids;
xii. une l'huile essentielle de *Citrus reticulata,* de préférence à une concentration de 0,1 à 0,4% en poids, de préférence à une concentration de 0,2% en poids; et
xiii. de l'acétate d'α-tocophérol (vitamine E), de préférence à une concentration de 2,5 à 7,5% en poids, de préférence à une concentration de 5% en poids;

Les deux ingrédients clés de ce masque sont l'alchémille (*Alchemilla vulgaris* sp. *Xanthochlora*) et la vitamine E (acétate d'α-tocophérol). De préférence, cette plante est cueillie à la main et séchée manuellement. Bien qu'il s'agisse d'un processus laborieux, il permet de maintenir les propriétés de la plante à leur valeur maximale. Cette plante a une fonction anti-inflammatoire et joue un rôle dans la production de la matrice extra-cellulaire et possède des propriétés procollagène. Elle possède également une fonction d'inhibition de la tyrasinase et provoque un ressenti de fraîcheur.

Le masque comprend de la vitamine E, de préférence à une concentration d'au moins 4% en poids, de préférence au moins 5% en poids.

La combinaison de l'alchémille avec la vitamine E, l'extrait de concombre, le beurre de karité et l'huile de mandarine fournit la richesse des composants clés pour réduire les rides, adoucir la peau, hydrater, réduire les inflammations ou les irritations, blanchir la peau et équilibrer le terrain psychologique. Ce dernier point est dû à l'huile de mandarine qui favorise un sommeil sain grâce la présence d'une concentration élevée de limonène et au λ-terpinène. De préférence, l'huile de mandarine utilisée comporte une concentration de limonène d'au moins 70% en poids.

Ce masque à la vitamine E est appliqué en combinaison avec un masque à l'argile consistant en une pâte comprenant de l'eau mélangée à une argile verte ayant un contenu en Silicium compris entre 60 et 75% en poids et comprenant également du fer, du calcium, du manganèse, de l'azote et du potassium. L'argile est l'eau sont de préférence utilisés dans un ratio de 1:4 à 1:6.

L'argile verte est de préférence pure et séchée au soleil. L'argile est particulièrement riche en silicium, qui est particulièrement important pour la peau afin de lui apporter fermeté et tonicité. En plus de sa richesse en fer, calcium, manganèse, azote et potassium, l'argile contient également de nombreux autres microéléments favorables à la peau.

La combinaison des différentes compositions du kit permet de créer les conditions qui favorisent l'absorption de ces éléments par la peau de la manière la plus efficace.

Le kit comprend également une crème comprenant
i. du monostéarate de glycérol, de préférence à une concentration de 2 à 5 % en poids, de préférence à une concentration de 3,5 % en poids;
ii. de l'alcool cétylique, de préférence à une concentration de 2 à 6 % en poids, de préférence à une concentration de 2,54 % en poids;
iii. des triglycérides saturés à chaînes moyennes, de préférence à une concentration de 4 à 7 % en poids, de préférence à une concentration de 5 % en poids;
iv. une composition comprenant de l'alcool cétéarylique et du sodium cétéaryl sulfate, de préférence à une concentration totale de 0,5 à 2,5 % en poids, de préférence à une concentration totale de 1 % en poids ;
v. une composition comprenant de l'huile de graines de *Ricinus Communis,* de l'huile de ricin hydrogénée et de la cire de *Copernicia Cerifera,* de préférence à une concentration totale de 7 à 15 % en poids, de préférence à une concentration totale de 10 % en poids;
vi. du palmitate cétylique, de préférence à une concentration de 2 à 5 % en poids, de préférence à une concentration de 3 % en poids;
vii. du glycérol, de préférence à une concentration de 2 à 6 % en poids, de préférence à une concentration de 4 % en poids;
viii. du propylène glycol, de préférence à une concentration de 5 à 12 % en poids, de préférence à une concentration de 7 % en poids;
ix. de l'eau distillée, de préférence à une concentration de 25 à 73 % en poids, de préférence à une concentration de 58 % en poids;
x. du sorbate de potassium, de préférence à une concentration de 0,05 à 0,3 % en poids, de préférence à une concentration de 0,15 % en poids;
xi. un extrait liquide d'*Alchemilla vulgaris,* de préférence à une concentration de 2 à 8 % en poids, de préférence à une concentration de 3,3 % en poids;
xii. de l'alcool benzylique, de préférence à une concentration de 0,001 à 0,1 % en poids, de préférence à une concentration de 0,01 % en poids;
xiii. une huile essentielle de *Citrus reticulata,* de préférence à une concentration de 0,05 à 0,6 % en poids, de préférence à une concentration de 0,3 % en poids;
xiv. de l'acétate d'α-tocophérol (vitamine E), de préférence à une concentration de 0,01 à 0,3 % en poids, de préférence à une concentration de 0,2 % en poids; et
xv. de l'oxyde de zinc, de préférence à une concentration de 1 à 4 % en poids, de préférence à une concentration de 2 % en poids

Cette crème est un cosmétique hydratant et équilibrant. Les principaux composants sont la vitamine E et l'alchémille, dont les propriétés ont été décrites précédemment. Pour assurer un effet durable, l'huile de mandarine est à nouveau utilisée pour stabiliser l'amélioration des émotions. Lorsqu'elle est utilisée seule, la crème offre des propriétés hydratantes et calmantes.

L'oxyde de zinc est avantageusement actif comme filtre UV.

Le kit de la présente invention peut également comporter, en plus des produits mentionnés ci-dessus, un sérum régénérant et/ou une lotion énergisante.

De préférence, le sérum régénérant comprend :
i. de l'acide hyaluronique, de préférence à une concentration de 0,1 à 0,3 % en poids, de préférence à une concentration de 0,25 % en poids;
ii. de l'extrait de concombre, de préférence dans le propylène glycol, de préférence à une concentration de 2,5 à 7,5 % en poids, de préférence à une concentration de 6 % en poids;
iii. de l'hydrolat de bleuet, de préférence à une concentration de 3 à 9 % en poids, de préférence à une concentration de 7 % en poids;
iv. du sorbate de potassium, de préférence à une concentration de 0,25 à 0,75 % en poids, de préférence à une concentration de 4 % en poids;
v. de l'huile essentielle de *Chamaemelum nobile* (camomille romaine) , de préférence à une concentration de 0,05 à 0,2 % en poids, de préférence à une concentration de 0.2 % en poids;
vi. du xanthane, de préférence à une concentration de 0,25 à 0,75 % en poids, de préférence à une concentration de 0,15 % en poids; et
vii. de l'eau distillée, de préférence à une concentration de 83,7 à 87,5 % en poids, de préférence à une concentration de 86 % en poids.

De préférence, la lotion énergisante comprend :
i. de l'eau de rose concentrée, de préférence à une concentration de 1à 2% en poids, de préférence à une concentration de 1,2% en poids;
ii. de l'eau de fleurs d'oranger concentrée, de préférence à une concentration de 2,5 à 7,5% en poids, de préférence à 5,1% en poids;
iii. de l'eau distillée, de préférence à une concentration de 82 à 96% en poids, de préférence à une concentration de 88% en poids;
iv. de l'extrait de pépins de pamplemousse, de préférence à une concentration de 1,5 à 3,2% en poids, de préférence à une concentration de 2,1% en poids;
v. du sorbate de potassium, de préférence à une concentration de 1,5 à 2,5% en poids, de préférence à une concentration de 1,9% en poids; et
vi. du benzoate de sodium, de préférence à une concentration de 1,5 à 2,5% en poids, de préférence à une concentration de 1,7% en poids.

L'invention porte également sur un procédé pour le raffermissement de la peau, pour l'amélioration de l'apparence de la peau, pour l'amélioration de l'hydratation de la peau, pour la réduction des taches foncées, telles que les lentigines solaires, taches de rousseur et taches de vieillissement (taches brunes), pour la réduction de la ptose de la peau, pour la réduction des rides, pour le remplissage des sillons naso-géniens, pour l'amélioration des émotions, pour l'amélioration de l'équilibre psycho-émotionnel, pour la réduction de l'anxiété et/ou pour la réduction de l'irritabilité d'un individu. La ptose est de préférence la ptose de la peau du bas du visage. Les rides sont de préférences des rides superficielles.

De préférence l'individu est dans la phase de péri-ménopause et/ou est âgé de 45 à 65 ans. L'individu peut aussi bien être une femme qu'un homme.

Ce procédé comprend l'application successive de toutes les compositions contenues dans le kit de l'invention décrit plus haut. Les différentes étapes du procédé permettent une optimisation de l'efficacité du kit, par la définition de techniques d'application spécifiques pour chaque composition présente dans le kit. Ces technique spécifiques et l'ordre d'application des différentes compositions permettent cette optimisation. Ainsi les étapes a) à i) du procédé sont de préférence effectuées successivement dans l'ordre spécifique mentionné ci-dessus.

Dans une variante préférée du kit de l'invention, ce kit comprend, en plus des compositions citées plus haut, une notice indiquant la technique optimale d'application des compositions du kit et comprenant la description des différentes étapes du procédé tel que décrit ci-dessous.

La première étape du procédé comprend l'application sur la peau du lait démaquillant et nourrissant. Ce lait est de préférence appliqué sur les deux mains de la personne qui l'applique sur le sujet et un massage d'une à deux minutes est effectué afin de distribuer le lait de manière homogène sur la peau. Le lait peut rester sur la peau et il n'est pas nécessaire de l'enlever avant l'application de la composition suivante.

La seconde étape essentielle du procédé est l'application du peeling. De préférence le peeling est appliqué par des mouvements circulaires avec les mains pendant 2 à 3 minutes. Il est essentiel d'enlever toutes traces du peeling après application, de préférence avec une serviette humide chaude.

Dans une variante préférée de l'invention, une solution énergisante est appliquée entre le lait démaquillant et nourrissant et le peeling.

Après le peeling, c'est la composition pour les capillaires qui est appliquée. La dose de préférence est de 2 gouttes. Le produit est de préférence distribué de manière uniforme sur l'ensemble du visage, à l'exception des lèvres et du contour supérieur des yeux. Il peut être appliqué sur la partie inférieure du contour des yeux (paupière inférieure). Afin d'assurer une efficacité optimale, cette composition est de préférence sur les zones problématiques, telles que les taches rouges et les zones enflammées. Ainsi, la composition agit plus longuement sur ces zones.

Ensuite on applique le masque riche en vitamine E, qui est de préférence distribué de manière uniforme sur l'ensemble du visage, y compris sur le contour des yeux et les lèvres. De préférence, on applique une couche de gaze sur le masque, cette gaze étant de préférence maintenue avec des lanières par-dessus la tête. Cela a l'avantage d'étirer la peau du visage.

Le masque à l'argile est ensuite appliqué, de préférence immédiatement après le masque à la vitamine E. Le masque est de préférence préparé juste avant application en mélangeant l'argile avec de l'eau pour former une pâte. La pâte est appliquée sur une fine couche de gaze. Il est recommandé d'éviter les zones des yeux, des paupières et des lèvres. Le masque est typiquement appliqué durant une période pose de 15 à 25 minutes, de préférence 20 minutes. Si le masque sèche durant la pose et tend à coller sur le visage, il est de préférence réhumidifié en appliquant un doigt mouillé sur les zones sèches. Le masque est ensuite enlevé, par exemple en soulevant la gaze lentement. Les résidus du masque sont de préférence enlevés en essuyant la peau à l'aide d'une serviette humide et chaude.

La dernière étape essentielle du procédé de l'invention est l'application de la crème. Celle-ci est de préférence appliquée en petite quantité par des tapotements légers. Il n'est pas nécessaire d'enlever le crème du visage après application.

Dans une variante préférée du procédé de l'invention, un sérum régénérant est appliqué entre le masque à l'argile et la crème. De préférence, 5 gouttes de sérum sont appliquées par des mouvements circulaires avec les mains jusqu'à pénétration complète.

Dans une autre variante préférée de l'invention, la lotion énergisante est appliquée après l'application du lait démaquillant et hydratant et avant l'application du peeling. De préférence, cette lotion est appliquée sur la peau à l'aide d'un coton.

Il est recommandé de ne pas exposer la peau au soleil direct durant les 8 à 12 heures après l'application du soin.

Le procédé peut typiquement être exécuté par un professionnel de la cosmétique ou de l'esthétique, dans une clinique ou un centre de bien-être.

### Exemples

L'efficacité d'un soin cosmétique consistant en un kit de compositions selon la présente invention a été testé.

### Synopsis

La présente étude a pour objectif de vérifier sous contrôle dermatologique l'acceptabilité par la peau des compositions du soin et d'évaluer les propriétés biomécaniques de la peau sur un panel de sujets humains sains après une seule application dans les conditions recommandées pour les cliniques et salons de beauté et d'esthétique, avant et après application. Les propriétés suivantes ont été testées :
- l'efficacité anti-rides par notation clinique des rides de la patte d'oie
- l'efficacité sur la réduction de la ptose de la partie inférieure du visage
- l'efficacité sur le remplissage des sillons naso-géniens
- l'efficacité sur l'intensité des taches foncées et taches de vieillissement (taches brunes) par notation clinique
- l'efficacité par la notation clinique des cernes et des poches des yeux (gonflement)
- les effets émotionnels subjectifs induits par l'application du produit.

L'étude a porté sur 20 sujets.

Les critères d'inclusions spécifiques étaient les suivants :
- Âge compris entre 45 et 65 ;
- Sexe féminin ;
- Phototype (Fitzpatrick) : II à IV ;
- Tous types de peau ;
- Présence de rides sur le visage (région de la patte d'oie) d'un degré minimum de 2 ;
- Ptose de la partie inférieure du visage, d'une importance correspondant à un degré de 1 à 5, telle qu'évaluée sur une échelle à 6 degrés ;
- Présence de cernes et de poches sous les yeux et au moins trois éléments de pigmentation sur le visage (lentigines solaires, taches de rousseur et taches de vieillissement (taches brunes)) ;
- Usage régulier de produits cosmétiques pour le visage ;
- Sommeil nocturne interrompu et ressenti d'anxiété et d'irritabilité ; et
- Consentement du sujet à porter l'équipement fourni et à suivre les instructions des investigateurs durant l'étude.
- Présence de sillons naso-géniens

Les critères d'exclusion spécifiques étaient les suivants :
- Présence d'allergie connue à des produits similaires aux produits testés ; et
- Historique personnel ou familiale d'atopie.

La méthodologie d'application était la suivante : application unique au centre par le technicien dans les conditions d'utilisation prévues par le moniteur de l'étude.

Les compositions appliquées, les zones de la peau sur lesquelles lesdites composition ont été appliquées, ainsi que les techniques d'application utilisées sont résumées dans la [Table 1] ci-dessous :

**[Table 1]**

| **Composition** | **Zone d'application** | **Technique d'application** |
|---|---|---|
| Lait démaquillant et nourrissant | Visage | Application sur les mains (2 pompes) et massage doux sur le visage. Le produit n'est pas retiré après application. |
| Lotion énergisante | Visage | Application sur un coton, puis application sur le visage en pressant doucement le coton sur le visage. Le produit n'est pas retiré après application. |
| Peeling | Visage (avec exclusion de la zone des yeux) et cou | Application sur les mains puis application sur le visage par des mouvements circulaires des mains pendant 2 à 3 minutes. Le produit est ensuite retiré délicatement avec une serviette chaude humide. Le produit doit être très bien lavé sans laisser de trace. |
| Composition pour les capillaires | Visage (y-compris la partie inférieure du contour des yeux, mais pas sur la partie supérieure du contour des yeux et sur les lèvres) | Deux gouttes de produit sont réparties sur l'ensemble du visage, y compris la partie inférieure du contour des yeux (paupière inférieure). Application en premier sur les zones à problèmes (taches rouges, inflammation). Le produit n'est pas retiré après application. |
| Masque à la vitamine E | Visage, cou et décolleté | Application uniforme sur l'ensemble du visage. Par-dessus le masque, une gaze est appliquée sur tout le visage et elle est serrée avec 2 bandes au-dessus de la tête pour étirer la peau du visage. Des orifices pour les yeux et le nez seront créés. Le produit n'est pas retiré après application. |
| Maque à l'argile | Visage | L'argile est mélangée avec de l'eau pour former une pâte. La pâte obtenue est appliquée sur une gaze fine. La zone des yeux, des sourcils et des lèvres est évitée. Le masque est laissé en place durant 20 minutes. Il est retiré ensuite en enroulant le tissu de bas en haut. Si le tissu devient trop sec et colle au visage, il est réhumidifié avec les doigts. A la fin de la durée de pose, il est retiré lentement. Le visage est ensuite essuyé avec une serviette chaude et humide. |
| Sérum régénérant | Visage | Application de 5 gouttes par des mouvements circulaires des mains sur l'ensemble du visage, y compris le contour des yeux et les lèvres. |
| Crème | Visage | Application d'une petite quantité de produit à l'aide de tapotements légers. Le produit n'est pas retiré après application. |

L'exposition au soleil est évitée durant les 8 à 12 heures suivant l'application du soin.

La composition des produits appliqués et décrite dans les tables 2 à 8 ci-dessous.

**[Table 2]: composition du lait démaquillant et nourrissant**

| **Ingrédient** | **Concentration [% en poids]** |
|---|---|
| Monostéarate de glycérol | 7,00 |
| Alcool cétylique | 1,40 |
| Diméticone | 0,30 |
| Acétate d'α-tocophérol | 0,05 |
| Triglyérides saturés à chaîne moyenne | 8,40 |
| Sorbate de potassium | 0,03 |
| EDTA | 0,06 |
| Propylèneglycol | 1,25 |
| Carbopol | 0,30 |
| Huile essentielle de mandarine verte | 0,25 |
| Hydroxyde de sodium | 1,00 |
| Eau | 79.96 |

**[Table 3]: composition de la lotion énergisante**

| **Ingrédient** | **Concentration [% en poids]** |
|---|---|
| Eau de rose concentrée | 1,20 |
| Eau de fleurs d'oranger concentrée | 5,10 |
| Eau distillée | 88.00 |
| Extrait de pépins de pamplemousse | 2,10 |
| Sorbate de potassium | 1,90 |
| Benzoate de sodium | 1,70 |

**[Table 4]: composition du peeling (pH 2,7)**

| **Ingrédient** | **Concentration [% en poids]** |
|---|---|
| Eau | 61,5 |
| Monostéarate de glycérol | 6,40 |
| Agent exfoliant contenant du polyéthylène et du stéarate de zinc (Gotalene^{®} bright) | 3,00 |
| Composition comprenant de l'alcool cétéarylique, du sodium cétéaryl sulfate (Lanette^{®} SX 6) | 3,00 |
| Triglycérides saturés à chaines moyennes | 6,02 |
| Alcool cétylique | 5,66 |
| Sorbate de potassium | 0,25 |
| Propylène glycol | 10,00 |
| Acid salicylique | 3,60 |
| Xanthane | 0,17 |
| Huile de feuilles de *Mentha arvensis* | 0,20 |
| Huile de *Cymbopogon martini* | 0,20 |

**[Table 5]: composition du produit pour le traitement des capillaires**

| **Ingrédient** | **Concentration [% en poids]** |
|---|---|
| Huile de *Rosa moscata* | 87,00 |
| Huile de bois de *Cupressus funebris* | 7,74 |
| Huile de fleur de *Chamomilla recutita* | 4,05 |
| Huile de fleur d'*Helichrysum arenarium* | 0,60 |
| Huile de Géranium bourbon | 0,60 |
| De l'acétate d'α-tocophérol (vitamine E) | 0,01 |

**[Table 6]: composition du masque à la vitamine E**

| **Ingrédient** | **Concentration [% en poids]** |
|---|---|
| Eau distillée | 34,65 |
| une composition comprenant de l'huile de graines de *Ricinus Communis,* de l'huile de ricin hydrogénée et de la cire de *Copernicia Cerifera;* | 15,00 |
| Monostéarate de glycérol 40-55 | 4,00 |
| Extrait de concombre | 8,00 |
| Beurre de karité | 2,50 |
| Composition comprenant de l'alcool cétéarylique et du sodium cétéaryl sulfate (Lanette N) | 4,00 |
| Triglycérides saturés à chaîne moyenne | 7.50 |
| Alcool cétylique | 6,00 |
| Sorbate de potassium | 0,15 |
| Propylène glycol | 5,00 |
| Extrait liquide d'*Alchemilla vulgaris* | 8,00 |
| Acétate d'α-tocophérol (Vitamine E) | 5,00 |
| Huile de peau de *Citrus reticulata* | 0,20 |

**[Table 7]: composition du sérum régénérant**

| **Ingrédient** | **Concentration [% en poids]** |
|---|---|
| Acide hyaluronique (Hyacare 50 Rahn) | 0,25 |
| Extrait de concombre (Herbasol) | 6,00 |
| Hydrolat de bleuet | 7,00 |
| Sorbate de potassium | 0,40 |
| Huile essentielle de *Chamaemelum nobile* | 0,20 |
| Xanthane | 0,15 |
| Eau distillée | 86,00 |

**[Table 8]: composition de la crème**

| **Ingrédient** | **Concentration [% en poids]** |
|---|---|
| Eau | 58,00 |
| Monostéarate de glycérol | 3,50 |
| Propylène glycol | 7,00 |
| Composition comprenant de l'alcool cétéarylique et du sodium cétéaryl sulfate | 1,00 |
| glycérol | 4,00 |
| triglycérides saturés à chaînes moyennes | 5,00 |
| Alcool cétylique | 2,54 |
| Sorbate de potassium | 0,15 |
| Composition comprenant de l'huile de graines de *Ricinus Communis,* de l'huile de ricin hydrogénée et de la cire de *Copernicia Cerifera* | 10,00 |
| Oxyde zinc | 2,00 |
| Extrait liquide d'alchemille | 3,30 |
| Acétate d'α-tocophérol | 0,20 |
| Huile essentielle de *Citrus reticulata* | 0,30 |
| Alcool benzylique | 0,01 |
| Palmitate cétylique | 3,00 |

Le masque à l'argile comprend de l'argile verte et de l'eau dans un rapport de 1 à 5. L'argile a un contenu en ayant un contenu en silicium d'environ 70% et comprend également du fer, du calcium, du manganèse, de l'azote et du potassium.

La tolérance du soin par la peau a été évaluée comme suit :
- un examen cutané des zones expérimentales par l'investigateur :
   ∘ avant l'application des produits (J₁/T₀)
   ∘ puis après l'application des produits immédiatement après application (J₁/ T_{immediat}) et 10 - 15 minutes après l'application du soin. (J₁/T₁₀₋₁₅)
- l'analyse des sensations d'inconfort rapportées directement par les sujets testés à l'investigateur/sous-investigateur ou par le technicien, au cours de l'étude.
- Analyse descriptive - Pourcentage de sujets réactifs.

L'évaluation de l'efficacité anti-ride a été effectuée par notation clinique sur la zone de la patte d'oie en comparant les résultats obtenus aux temps expérimentaux définis avec la ligne de base (J₁/T₀) (analyse statistique) :
- avant l'application des produits (J₁/T₀)
- puis après l'application des produits immédiatement après application (J₁/T_{immediat}) et 10-15 minutes après l'application du soin (J1/T₁₀₋₁₅).

L'évaluation de l'efficacité sur la ptose de la partie inférieure du visage a été effectuée par notation clinique, à la fois par l'investigateur et par le sujet du test, en comparant les résultats obtenus aux temps expérimentaux définis avec la ligne de base (J₁/T₀) (analyse statistique) :
- avant l'application des produits (J₁/T₀)
- puis après l'application des produits immédiatement après application (J₁/ T_{immediat}) et 10 - 15 minutes après l'application du soin. (J₁/T₁₀₋₁₅)

L'évaluation de l'efficacité en ce qui concerne les cernes et les poches sous les yeux a été effectuée par notation clinique en comparant les résultats obtenus aux temps expérimentaux définis avec la ligne de base (J₁/T₀) (analyse statistique) :
- avant l'application des produits (J₁/T₀)
- puis après l'application des produits immédiatement après application application (J₁/ T_{immediat}) et 10 - 15 minutes après l'application du soin. (J₁/T₁₀₋₁₅)

L'évaluation de l'efficacité sur les éléments pigmentaires a été effectuée par notation clinique sur l'intensité de ces éléments pigmentaires en comparant les résultats obtenus aux temps expérimentaux définis avec la ligne de base (J₁/T₀) (analyse statistique) :
- avant l'application des produits (J₁/T₀)
- puis après l'application des produits immédiatement après application (J₁/ T_{immediat}) et 10 - 15 minutes après l'application du soin. (J₁/T₁₀₋₁₅)

L'appréciation des effets émotionnels subjectifs induits par l'application des produits a été évalué après analyse d'un questionnaire adapté aux produits expérimentaux, élaboré par l'investigateur et rempli par chaque sujet testé après l'application des produits immédiatement après application application (J₁/ T_{immediat}) et 10 - 15 minutes après l'application du soin. (J₁/T₁₀₋₁₅). Le paramètre d'évaluation était le score d'appréciation, soit le pourcentage de sujets satisfaits.

### Résultats

**[Table 9]**

| | Sujets concernés | Date et raisons |
|---|---|---|
| Retraits | aucun | non applicable |
| exclusion | aucun | non applicable |
| Sujets pour lesquels des déviations majeures ont été observées | 0 | |
| Sujets valables | 20 | |

Aucun écart concernant les critères de non-inclusion n'a été constaté à l'inclusion.

Les caractéristiques typologiques individuelles des sujets du test sont rapportées dans le rapport final, et récapitulées ci-dessous :

**[Table 10]**

| **Âge (années)** | **Sujets inclus** |
|---|---|
| **Minimum** | 45 |
| **Maximum** | 63 |
| **Moyenne** | 55 |
| **Médiane** | 57 |

**[Table 11]**

| | **Sujets inclus** | |
|---|---|---|
| **Critère** | **Nombre** | **Pourcentage** |
| **Phototype** | | |
| II | 5 | 25% |
| III | 12 | 60% |
| IV | 3 | 15% |

| Sexe | | |
|---|---|---|
| Femme | 20 | 100% |

| **Type de peau sur le visage** | | |
|---|---|---|
| Normale | 6 | 30% |
| Sèche | 4 | 20% |
| Mixte sèche | 6 | 30% |
| Mixte grasse | 4 | 20% |

**[Table 12]**

| **Statut des rides dans la zone de la patte d'oie** | |
|---|---|
| **Minimum** | 2.00 |
| **Maximum** | 8.00 |
| **Moyenne** | 5.25 |

| **Statut de la ptose de la partie inférieure du visage** | |
|---|---|
| **Minimum** | 2.00 |
| **Maximum** | 4.00 |
| **Mean** | 3.05 |

### Acceptabilité du soin par la peau

Aucun signe clinique imputable au produit expérimental n'a été observé par l'investigateur de l'étude. Aucune sensation d'inconfort n'a été rapportée par les sujets au cours de l'étude.

### Évaluation de l'efficacité anti-rides par notation clinique dans la zone de la patte d'oie.

Les résultats de la notation clinique de l'efficacité anti-rides sont représentés dans la [Table 13]

**[Table 13]**

| | Nombre | Moyenne | Médiane | Déviation standard | Minimum | Maximum |
|---|---|---|---|---|---|---|
| J₁/T₀ | 20 | 5.25 | 5.50 | 1.65 | 2.00 | 8.00 |
| J₁/T₁ | 20 | 4.40 | 5.00 | 1.64 | 2.00 | 7.00 |
| J₁/T₁-J₁/T₀ | 20 | -.85 | -1.00 | 0.67 | -2.00 | 0.00 |
| Pourcentage de variation J₁/T₁ vs J₁/T₀ | 20 | -16.89 | -16.67 | 14.40 | -50.00 | 0.00 |

Comparaison de la notation au fil du temps est présentée dans la [Table 14] ci-dessous.

**[Table 14]**

| **Comparaison** | **Test** | **Valeur p** | **Interprétation statistique** |
|---|---|---|---|
| | | | *Significative si p<0.050* |
| J₁/T₀ *versus* J₁/T₁ | Test de Student (test t) | <0.001 | Réduction significative |

Interprétation : on observe une diminution significative du score des rides après l'application des produits par rapport à la ligne de base à J₁/T₀.

### Evaluation de la ptose de la partie inférieure du visage par notation clinique

La statistique descriptive de l'évaluation est présentée dans la [Table 15] ci-dessous.

**[Table 15]**

| | **N** | **Moyenne** | **Médiane** | **Déviation standard** | **Minimum** | **Maximum** |
|---|---|---|---|---|---|---|
| J₁/T₀ | 20 | 3.05 | 3.00 | 0.76 | 2.00 | 4.00 |
| J₁/T₁ | 20 | 2.20 | 2.50 | 0.89 | 1.00 | 3.00 |
| J₁/T₁- J₁/T₀ | 20 | -0.85 | -1.00 | 0.75 | -3.00 | 0.00 |
| % of variation J₁/T₁ vs J₁/T₀ | 20 | -28.33 | -25.00 | 23.48 | -75.00 | 0.00 |

Comparaison de la notation au fil du temps est présentée dans la [Table 16] ci-dessous.

**[Table 16]**

| **Comparaison** | **Test** | **Valeur p** | **Interprétation statistique** |
|---|---|---|---|
| | | | *Significative si p<0.050* |
| J₁/T₀ *versus* J₁/T₁ | Test des rangs signés de Wilcoxon | <0.001 | Réduction significative |

Interprétation : on observe une diminution significative du score de la ptose du bas du visage après l'application des produits du soin par rapport à la ligne de base à J₁/T₀.

### Evaluation du statut des cernes

La statistique descriptive de l'évaluation est présentée dans la [Table 17] ci-dessous.

**[Table 17]**

| | **N** | **Moyenne** | **Médiane** | **Déviation standard** | **Minimum** | **Maximum** |
|---|---|---|---|---|---|---|
| J₁/T₀ | 20 | 4.30 | 1.26 | 0.76 | 2.00 | 7.00 |
| J₁/T₁ | 20 | 3.40 | 1.35 | 0.89 | 1.00 | 6.00 |
| J₁/T₁- J₁/T₀ | 20 | -0.90 | -1.00 | 0.72 | -3.00 | 1.00 |
| % of variation J₁/T₁ vs J₁/T₀ | 20 | -20.76 | -25.00 | 22.42 | -75.00 | 50.00 |

Comparaison de la notation au fil du temps est présentée dans la [Table 18] ci-dessous.

**[Table 18]**

| **Comparaison** | **Test** | **Valeur p** | **Interprétation statistique** |
|---|---|---|---|
| | | | *Significative si p<0.050* |
| J₁/T₀ *versus* J₁/T₁ | Test de Student (test t) | <0.001 | Réduction significative |

Interprétation : on observe une diminution significative du score des cernes après l'application des produits du soin par rapport à la ligne de base à J₁/T₀.

### Evaluation du statut des poches sous les yeux

La statistique descriptive de l'évaluation est présentée dans la [Table 19] ci-dessous.

**[Table 19]**

| | **N** | **Moyenne** | **Médiane** | **Déviation standard** | **Minimum** | **Maximum** |
|---|---|---|---|---|---|---|
| J₁/T₀ | 20 | 4.65 | 4.50 | 1.60 | 2.00 | 7.00 |
| J₁/T₁ | 20 | 3.90 | 4.00 | 1.65 | 1.00 | 7.00 |
| J₁/T₁- J₁/T₀ | 20 | -0.75 | -1.00 | 0.72 | -2.00 | 0.00 |
| % of variation J₁/T₁ vs J₁/T₀ | 20 | -17.48 | -16.67 | 19.52 | -66.67 | 0.00 |

Comparaison de la notation au fil du temps est présentée dans la [Table 20] ci-dessous.

**[Table 20]**

| **Comparaison** | **Test** | **Valeur p** | **Interprétation statistique** |
|---|---|---|---|
| | | | *Significative si p<0.050* |
| J₁/T₀ *versus* J₁/T₁ | Test de Student (test t) | <0.001 | Réduction significative |

Interprétation : on observe une diminution significative du score des poches sous les yeux après l'application des produits du soin par rapport à la ligne de base à J₁/T₀.

### Evaluation de l'intensité des taches foncées (lentigines solaires, taches de rousseur et taches de vieillissement (taches brunes))

La statistique descriptive de l'évaluation est présentée dans la [Table 21] ci-dessous.

**[Table 21]**

| | **N** | **Moyenne** | **Médiane** | **Déviation standard** | **Minimum** | **Maximum** |
|---|---|---|---|---|---|---|
| J₁/T₀ | 20 | 4.05 | 4.00 | 1.39 | 2.00 | 7.00 |
| J₁/T₁ | 20 | 3.10 | 2.50 | 1.41 | 1.00 | 6.00 |
| J₁/T₁- J₁/T₀ | 20 | -0.95 | -1.00 | 0.69 | -3.00 | 0.00 |
| % of variation J₁/T₁ vs J₁/T₀ | 20 | -24.63 | -22.50 | 17.35 | -60.00 | 0.00 |

Comparaison de la notation au fil du temps est présentée dans la [Table 22] ci-dessous.

**[Table 22]**

| **Comparaison** | **Test** | **Valeur p** | **Interprétation statistique** |
|---|---|---|---|
| | | | *Significative si p<0.050* |
| J₁/T₀ *versus* J₁/T₁ | Test de Student (test t) | <0.001 | Réduction significative |

Interprétation : on observe une diminution significative de l'intensité des taches foncées (lentigines solaires, taches de rousseur et taches de vieillissement (taches brunes)) après l'application des produits du soin par rapport à la ligne de base à J₁/T₀.

### Statut des problèmes cutanés et émotionnels par les sujets eux-mêmes au J1/T0 avant application du soin

Les résultats de l'évaluation sont présentés dans les tables 23 à 41 ci-dessous

**[Table 23]**

| **I.** | **Comment décririez-vous votre type de peau** | | |
|---|---|---|---|
| Nombre total | 20 | Nombre | Pourcentage |
| Normal | | 4 | 20% |
| Grasse | | 0 | 0% |
| Sèche | | 4 | 20% |
| Mixte grasse | | 5 | 25% |
| Mixte sèche | | 7 | 35% |

**[Table 24]**

| **II.1.** | **Comment décririez-vous vos problèmes de peau : état des boutons** | | |
|---|---|---|---|
| Nombre total | 20 | Nombre | Pourcentage |
| Score de 0 à 3 | | 20 | 100% |
| Score de 4 à 5 | | 0 | 0% |
| Score de 6 à 9 | | 0 | 0% |
| Moyenne | | 0.30 | |
| Déviation standard | | 0.73 | |

**[Table 25]**

| **II.2.** | **Comment décririez-vous vos problèmes de peau : état des taches de rousseur** | | |
|---|---|---|---|
| Nombre total | 20 | Nombre | Pourcentage |
| Score de 0 à 3 | | 0 | 0% |
| Score de 4 à 5 | | 7 | 35% |
| Score de 6 à 9 | | 13 | 65% |
| Moyenne | | 5.90 | |
| Déviation standard | | 0.85 | |

**[Table 26]**

| **II.3.** | **Comment décririez-vous vos problèmes de peau : état des éléments pigmentaires** | | |
|---|---|---|---|
| Nombre total | 20 | Nombre | Pourcentage |
| Score de 0 à 3 | | 6 | 30% |
| Score de 4 à 5 | | 8 | 40% |
| Score de 6 à 9 | | 6 | 30% |
| Moyenne | | 4.60 | |
| Déviation standard | | 1.67 | |

**[Table 27]**

| **II.4.** | **Comment décririez-vous vos problèmes de peau : manque de fermeté du bas du visage** | | |
|---|---|---|---|
| Nombre total | 20 | Nombre | Pourcentage |
| Score de 0 à 3 | | 6 | 30% |
| Score de 4 à 5 | | 6 | 30% |
| Score de 6 à 9 | | 8 | 40% |
| Moyenne | | 4.80 | |
| Déviation standard | | 2.04 | |

**[Table 28]**

| **II.5.** | **Comment décririez-vous vos problèmes de peau : manque de fermeté des joues** | | |
|---|---|---|---|
| Nombre total | 20 | Nombre | Pourcentage |
| Score de 0 à 3 | | 6 | 30% |
| Score de 4 à 5 | | 6 | 30% |
| Score de 6 à 9 | | 8 | 40% |
| Moyenne | | 4.65 | |
| Déviation standard | | 1.93 | |

**[Table 29]**

| **II.6.** | **Comment décririez-vous vos problèmes de peau : manque de fermeté plis nasaux** | | |
|---|---|---|---|
| Nombre total | 20 | Nombre | Pourcentage |
| Score de 0 à 3 | | 5 | 25% |
| Score de 4 à 5 | | 7 | 35% |
| Score de 6 à 9 | | 8 | 40% |
| Moyenne | | 5.00 | |
| Déviation standard | | 2.08 | |

**[Table 30]**

| **II.7.** | **Comment décririez-vous vos problèmes de peau : rides sur la partie supérieure du visage** | | |
|---|---|---|---|
| Nombre total | 20 | Nombre | Pourcentage |
| Score de 0 à 3 | | 4 | 20% |
| Score de 4 à 5 | | 8 | 40% |
| Score de 6 à 9 | | 8 | 40% |
| Moyenne | | 5.05 | |
| Déviation standard | | 1.82 | |

**[Table 31]**

| **II.8.** | **Comment décririez-vous vos problèmes de peau : rides sur la partie inférieure du visage** | | |
|---|---|---|---|
| Nombre total | 20 | Nombre | Pourcentage |
| Score de 0 à 3 | | 5 | 25% |
| Score de 4 à 5 | | 5 | 25% |
| Score de 6 à 9 | | 10 | 50% |
| Moyenne | | 5.20 | |
| Déviation standard | | 1.91 | |

**[Table 32]**

| **II.9.** | **Comment décririez-vous vos problèmes de peau : rides autour des yeux** | | |
|---|---|---|---|
| Nombre total | 20 | Nombre | Pourcentage |
| Score de 0 à 3 | | 2 | 10% |
| Score de 4 à 5 | | 7 | 35% |
| Score de 6 à 9 | | 11 | 55% |
| Moyenne | | 5.75 | |
| Déviation standard | | 1.71 | |

**[Table 33]**

| **II.10.** | **Comment décririez-vous vos problèmes de peau : rougeur** | | |
|---|---|---|---|
| Nombre total | 20 | Nombre | Pourcentage |
| Score de 0 à 3 | | 8 | 40% |
| Score de 4 à 5 | | 9 | 45% |
| Score de 6 à 9 | | 3 | 15% |
| Moyenne | | 3.25 | |
| Déviation standard | | 2.40 | |

**[Table 34]**

| **II.11.** | **Comment décririez-vous vos problèmes de peau : irritation et sensibilité après une exposition au soleil** | | |
|---|---|---|---|
| Nombre total | 20 | Nombre | Pourcentage |
| Score de 0 à 3 | | 13 | 65% |
| Score de 4 à 5 | | 1 | 5% |
| Score de 6 à 9 | | 6 | 30% |
| Moyenne | | 2.85 | |
| Déviation standard | | 2.87 | |

**[Table 35]**

| **II.12.** | **Comment décririez-vous vos problèmes de peau : irritation et sensibilité au produits cosmétiques** | | |
|---|---|---|---|
| Nombre total | 20 | Nombre | Pourcentage |
| Score de 0 à 3 | | 19 | 95% |
| Score de 4 à 5 | | 0 | 0% |
| Score de 6 à 9 | | 1 | 5% |
| Moyenne | | 0.3 | |
| Déviation standard | | 1.34 | |

**[Table 36]**

| **III.1.** | **Comment décririez-vous vos problèmes émotionnels et sensitifs : échauffement** | | |
|---|---|---|---|
| Nombre total | 20 | Nombre | Pourcentage |
| Score de 0 à 3 | | 16 | 80% |
| Score de 4 à 5 | | 4 | 20% |
| Score de 6 à 9 | | 0 | 0% |
| Moyenne | | 2.05 | |
| Déviation standard | | 1.57 | |

**[Table 37]**

| **III.2.** | **Comment décririez-vous vos problèmes émotionnels et sensitifs : irritabilité** | | |
|---|---|---|---|
| Nombre total | 20 | Nombre | Pourcentage |
| Score de 0 à 3 | | 0 | 0% |
| Score de 4 à 5 | | 4 | 20% |
| Score de 6 à 9 | | 16 | 80% |
| Moyenne | | 6.05 | |
| Déviation standard | | 0.83 | |

**[Table 38]**

| **III.3.** | **Comment décririez-vous vos problèmes émotionnels et sensitifs** : **anxiété** | | |
|---|---|---|---|
| Nombre total | 20 | Nombre | Pourcentage |
| Score de 0 à 3 | | 0 | 0% |
| Score de 4 à 5 | | 5 | 25% |
| Score de 6 à 9 | | 15 | 75% |
| Moyenne | | 6.20 | |
| Déviation standard | | 0.95 | |

**[Table 39]**

| **III.4.** | **Comment décririez-vous vos problèmes émotionnels et sensitifs : troubles du sommeil** | | |
|---|---|---|---|
| Nombre total | 20 | Nombre | Pourcentage |
| Score de 0 à 3 | | 0 | 0% |
| Score de 4 à 5 | | 4 | 20% |
| Score de 6 à 9 | | 16 | 80% |
| Moyenne | | 6.05 | |
| Déviation standard | | 0.69 | |

**[Table 40]**

| **III.5.** | **Comment décririez-vous vos problèmes émotionnels et sensitifs** : **fatigue le soir ou durant la journée** | | |
|---|---|---|---|
| Nombre total | 20 | Nombre | Pourcentage |
| Score de 0 à 3 | | 2 | 10% |
| Score de 4 à 5 | | 12 | 60% |
| Score de 6 à 9 | | 6 | 30% |
| Moyenne | | 4.95 | |
| Déviation standard | | 1.05 | |

**[Table 41]**

| **III.5.** | **Comment décririez-vous vos problèmes émotionnels et sensitifs** : **manque d'énergie** | | |
|---|---|---|---|
| Nombre total | 20 | Nombre | Pourcentage |
| Score de 0 à 3 | | 4 | 20% |
| Score de 4 à 5 | | 11 | 55% |
| Score de 6 à 9 | | 5 | 25% |
| Moyenne | | 4.50 | |
| Déviation standard | | 1.19 | |

### QUESTIONNAIRE J₁/T_{durant-application}

Les résultats de l'évaluation sont présentés dans les tables 42 à 46 ci-dessous

**[Table 42]**

| **Q1.** | **Comment décririez-vous ce que vous avez ressenti durant l'application du soin : effet calmant** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.80 | |
| Déviation standard | | 0.41 | |

**[Table 43]**

| **Q2.** | **Comment décririez-vous ce que vous avez ressenti durant l'application du soin : sensation de fraîcheur** | | |
|---|---|---|---|
| Nombre total | 20 | Nombre | Pourcentage |
| Score de 0 à 3 | | 1 | 5% |
| Score de 4 à 5 | | 0 | 0% |
| Score de 6 à 9 | | 19 | 95% |
| Moyenne | | 8.85 | |
| Déviation standard | | 1.79 | |

**[Table 44]**

| **Q3.** | **Comment décririez-vous ce que vous avez ressenti durant l'application du soin : envie de dormir** | | |
|---|---|---|---|
| Nombre total | 20 | Nombre | Pourcentage |
| Score de 0 à 3 | | 2 | 10% |
| Score de 4 à 5 | | 4 | 20% |
| Score de 6 à 9 | | 14 | 70% |
| Moyenne | | 6.80 | |
| Déviation standard | | 2.38 | |

**[Table 45]**

| **Q4.** | **Comment décririez-vous ce que vous avez ressenti durant l'application du soin : odeur plaisante** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.25 | |
| Déviation standard | | 0.97 | |

**[Table 46]**

| **Q5.** | **Comment décririez-vous ce que vous avez ressenti durant l'application du soin : sensation de relaxation** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 9.00 | |
| Déviation standard | | 0.00 | |

### QUESTIONNAIRE J₁/T_{après-application}

Les résultats de l'évaluation sont présentés dans les tables 47 à 64 ci-dessous

**[Table 47]**

| **Q1.** | **Comment décririez-vous ce que vous avez ressenti après l'application du soin : sensation de bien-être** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 9.00 | |
| Déviation standard | | 0.00 | |

**[Table 48]**

| **Q2.** | **Comment décririez-vous ce que vous avez ressenti après l'application du soin : effet calmant** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.95 | |
| Déviation standard | | 0.22 | |

**[Table 49]**

| **Q3.** | **Comment décririez-vous ce que vous avez ressenti après l'application du soin : sensation de fraîcheur** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 9.00 | |
| Déviation standard | | 0.00 | |

**[Table 50]**

| **Q4.** | **Comment décririez-vous ce que vous avez ressenti après l'application du soin : sensation d'énergie** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.40 | |
| Déviation standard | | 0.99 | |

**[Table 51]**

| **Q5.** | **Comment décririez-vous ce que vous avez ressenti après l'application du soin : envie de dormir** | | |
|---|---|---|---|
| Nombre total | 20 | Nombre | Pourcentage |
| Score de 0 à 3 | | 1 | 5% |
| Score de 4 à 5 | | 3 | 15% |
| Score de 6 à 9 | | 16 | 80% |
| Moyenne | | 7.60 | |
| Déviation standard | | 2.21 | |

**[Table 52]**

| **Q6.** | **Comment décririez-vous ce que vous avez ressenti après l'application du soin : sensation de joie** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 9.00 | |
| Déviation standard | | 0.00 | |

**[Table 53]**

| **Q7.** | **Comment décririez-vous ce que vous avez ressenti après l'application du soin : sensation de relaxation** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.90 | |
| Déviation standard | | 0.45 | |

**[Table 54]**

| **Q8.** | **Comment décririez-vous ce que vous avez ressenti après l'application du soin : la peau est visiblement repulpée** | | |
|---|---|---|---|
| Nombre total | 20 | Nombre | Pourcentage |
| Score de 0 à 3 | | 1 | 5% |
| Score de 4 à 5 | | 6 | 30% |
| Score de 6 à 9 | | 13 | 65% |
| Moyenne | | 6.10 | |
| Déviation standard | | 1.92 | |

**[Table 55]**

| **Q9.** | **Comment décririez-vous ce que vous avez ressenti après l'application du soin : la peau du visage est visiblement rajeunie** | | |
|---|---|---|---|
| Nombre total | 20 | Nombre | Pourcentage |
| Score de 0 à 3 | | 0 | 0% |
| Score de 4 à 5 | | 1 | 5% |
| Score de 6 à 9 | | 19 | 95% |
| Moyenne | | 8.00 | |
| Déviation standard | | 1.08 | |

**[Table 56]**

| **Q10.** | **Comment décririez-vous ce que vous avez ressenti après l'application du soin : la peau du cou est visiblement rajeunie** | | |
|---|---|---|---|
| Nombre total | 20 | Nombre | Pourcentage |
| Score de 0 à 3 | | 0 | 0% |
| Score de 4 à 5 | | 1 | 5% |
| Score de 6 à 9 | | 19 | 95% |
| Moyenne | | 8.00 | |
| Déviation standard | | 1.17 | |

**[Table 57]**

| **Q11.** | **Comment décririez-vous ce que vous avez ressenti après l'application du soin : la peau est nourrie** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.80 | |
| Déviation standard | | 0.41 | |

**[Table 58]**

| **Q12.** | **Comment décririez-vous ce que vous avez ressenti après l'application du soin : la peau est hydratée** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 8.95 | |
| Déviation standard | | 0.22 | |

**[Table 59]**

| **Q13.** | **Comment décririez-vous ce que vous avez ressenti après l'application du soin : la peau est apaisée** | | |
|---|---|---|---|
| Nombre total | 20 | Nombre | Pourcentage |
| Score de 0 à 3 | | 0 | 0% |
| Score de 4 à 5 | | 0 | 0% |
| Score de 6 à 9 | | 20 | 100% |
| Moyenne | | 9.00 | |
| Déviation standard | | 0.00 | |

**[Table 60]**

| **Q14.** | **Comment décririez-vous ce que vous avez ressenti après l'application du soin : moins de taches de rousseur visibles** | | |
|---|---|---|---|
| Nombre total | 20 | Nombre | Pourcentage |
| Score de 0 à 3 | | 15 | 75% |
| Score de 4 à 5 | | 1 | 5% |
| Score de 6 à 9 | | 4 | 20% |
| Moyenne | | 2.10 | |
| Déviation standard | | 3.45 | |

**[Table 61]**

| **Q15.** | **Comment décririez-vous ce que vous avez ressenti après l'application du soin : moins d'éléments pigmentaires visibles** | | |
|---|---|---|---|
| Nombre total | 20 | Nombre | Pourcentage |
| Score de 0 à 3 | | 14 | 70% |
| Score de 4 à 5 | | 3 | 15% |
| Score de 6 à 9 | | 3 | 15% |
| Moyenne | | 3.20 | |
| Déviation standard | | 2.55 | |

**[Table 62]**

| **Q16.** | **Comment décririez-vous ce que vous avez ressenti après l'application du soin : le manque de fermeté de la partie inférieure du visage est réduit** | | |
|---|---|---|---|
| Nombre total | 20 | Nombre | Pourcentage |
| Score de 0 à 3 | | 3 | 15% |
| Score de 4 à 5 | | 9 | 45% |
| Score de 6 à 9 | | 8 | 40% |
| Moyenne | | 5.45 | |
| Déviation standard | | 2.14 | |

**[Table 63]**

| **Q17.** | **Comment décririez-vous ce que vous avez ressenti après l'application du soin : le manque de fermeté de la partie supérieure du visage est réduit** | | |
|---|---|---|---|
| Nombre total | 20 | Nombre | Pourcentage |
| Score de 0 à 3 | | 3 | 15% |
| Score de 4 à 5 | | 10 | 50% |
| Score de 6 à 9 | | 7 | 35% |
| Moyenne | | 5.00 | |
| Déviation standard | | 2.25 | |

**[Table 64]**

| **Q18.** | **Comment décririez-vous ce que vous avez ressenti après l'application du soin : moins de comédons, de papules et de pustules** | | |
|---|---|---|---|
| Nombre total | 20 | Nombre | Pourcentage |
| Score de 0 à 3 | | 20 | 15% |
| Score de 4 à 5 | | 0 | 0% |
| Score de 6 à 9 | | 0 | 0% |
| Moyenne | | 0.00 | |
| Déviation standard | | 0.00 | |

### Conclusion

Dans les conditions expérimentales adoptées (application unique dans les conditions recommandées pour les cliniques et les salons de beauté et d'esthétique, par un panel de 20 sujets féminins, âgés de 45 à 63 ans, de phototype Il à IV, avec tous les types de peau du visage, avec une ptose de la partie inférieure du visage, ayant des rides sur le visage (zone de la patte d'oie), ayant des cernes et des poches sur les yeux (poches) et au moins trois éléments de pigmentation sur le visage (lentigines solaires, taches de rousseur et taches de vieillissement (taches brunes)), ayant un sommeil nocturne interrompu et étant anxieux et irrités), le soin composé des huit produits testé à démontré les effets suivants: une diminution significative des scores des rides, des ptoses, des cernes, des poches et de l'intensité des taches après l'application des produits par rapport à la situation de départ.

## Revendications

1. Kit de traitement cosmétique de la peau comprenant :
a) Un lait démaquillant et nourrissant ayant un pH de 5,3 et comprenant :
i. de l'eau;
ii. des triglycérides saturés à chaîne moyenne;
iii. du monostéarate de glycérol;
iv. du propylène glycol;
v. de l'alcool cétylique;
vi. du diméticone;
vii. de l'huile essentielle de mandarine verte;
viii. de l'acétate d'α-tocophérol (vitamine E);
ix. du carbopol;
x. de l'EDTA
xi. du sorbate de potassium; et
xii. de l'hydroxyde de sodium;
b) Une composition pour le traitement des capillaires comprenant
i. une huile végétale de *Rosa moschata;*
ii. une huile de *Cupressus sempervirens;*
iii. une huile de fleur d*'Chamaemelum nobile;*
iv. une huile de fleur d'*Helichrysum italicum;*
v. une huile de Géranium bourbon; et
vi. de l'acétate d'α-tocophérol;
c) Un peeling pour le visage ayant un pH de 2,7 à 2,8 et comprenant
i. du monostéarate de glycérol;
ii. de l'alcool cétylique;
iii. des triglycérides saturés à chaines moyennes;
iv. une composition comprenant de l'alcool cétéarylique et du sodium cétéaryl sulfate;
v. du propylène glycol;
vi. de l'eau;
vii. du sorbate de potassium;
viii. du xanthane,
ix. de l'acide salicylique;
x. un agent exfoliant contenant du polyéthylène et du stéarate de zinc;
xi. une huile essentielle de *Cymbopogon martini;* et
xii. une huile essentielle de menthe;
d) Un masque dont le pH est de 6,3 et comprenant
i. du monostéarate de glycérol;
ii. de l'alcool cétylique;
iii. des triglycérides à chaînes moyennes;
iv. une composition comprenant de l'alcool cétéarylique et du sodium cétéaryl sulfate;
v. une composition comprenant de l'huile de graines de *Ricinus Communis,* de l'huile de ricin hydrogénée et de la cire de *Copernicia Cerifera*
vi. du beurre de karité;
vii. de l'extrait de concombre;
viii. du propylène glycol;
ix. de l'eau distillée;
x. du sorbate de potassium;
xi. un l'extrait *d'Alchemilla vulgaris;*
xii. une l'huile essentielle de *Citrus reticulata;* et
xiii. de l'acétate d'α-tocophérol (vitamine E);
e) Un masque à l'argile consistant en une pâte comprenant de l'eau mélangée à une argile verte ayant un contenu en Silicium compris entre 60 et 75% en poids et comprenant également du fer, du calcium, du manganèse, de l'azote et du potassium;
f) Une crème comprenant
i. du monostéarate de glycérol;
ii. de l'alcool cétylique;
iii. des triglycérides saturés à chaînes moyennes;
une composition comprenant de l'alcool cétéarylique et du sodium cétéaryl sulfate;
v. une composition comprenant de l'huile de graines de *Ricinus Communis,* de l'huile de ricin hydrogénée et de la cire de *Copernicia Cerifera;*
vi. du palmitate cétylique;
vii. du glycérol;
viii. du propylène glycol;
ix. de l'eau distillée;
x. du sorbate de potassium;
xi. un extrait liquide d*'Alchemilla vulgaris;*
xii. de l'alcool benzylique;
xiii. une huile essentielle de *Citrus reticulata;*
xiv. de l'acétate d'α-tocophérol (vitamine E); et
xv. de l'oxyde de zinc

2. Un kit selon la revendication 1, comprenant également une lotion énergisante et/ou un sérum régénérant.

3. Un procédé pour le raffermissement de la peau, pour l'amélioration de l'apparence de la peau, pour l'amélioration de l'hydratation de la peau, pour la réduction des lentigines solaires, taches de rousseur et taches de vieillissement (taches brunes), pour la réduction de la ptose de la peau, pour la réduction des rides, pour le remplissage des sillons naso-géniens, pour l'amélioration des émotions, pour l'amélioration de l'équilibre psycho-émotionnel, pour la réduction de l'anxiété et/ou pour la réduction de l'irritabilité d'un individu comprenant
a) l'application sur la peau d'un lait démaquillant et nourrissant ayant un pH de 5,3 et comprenant:
i. de l'eau;
ii. des triglycérides saturés à chaîne moyenne;
iii. du monostéarate de glycérol,
iv. du propylène glycol,
v. de l'alcool cétylique;
vi. du diméticone;
vii. de l'huile essentielle de mandarine verte;
viii. de l'acétate d'α-tocophérol (vitamine E);
ix. du carbopol;
x. de l'EDTA
xi. du sorbate de potassium; et
xii. de l'hydroxyde de sodium; puis
b) l'application sur la peau d'un peeling pour le visage ayant un pH de 2,7 à 2,8 et comprenant
i. du monostéarate de glycérol;
ii. de l'alcool cétylique;
iii. des triglycérides à chaines moyennes;
iv. une composition comprenant de l'alcool cétéarylique et du sodium cétéaryl sulfate;
v. du propylène glycol;
vi. de l'eau;
vii. du sorbate de potassium;
viii. une solution de xanthane,
ix. de l'acide salicylique;
x. une micro-poudre exfoliante ayant une taille maximale de 350 µm;
xi. une huile essentielle de *Cymbopogon martini;* et
xii. une huile essentielle de menthe; puis
c) le rinçage complet du peeling appliqué dans l'étape c), de manière à ne laisser aucune trace dudit peeling sur la peau;
d) l'application sur la peau d'une composition pour le traitement des capillaires comprenant
i. une huile végétale de *Rosa moschata;*
ii. une huile de *Cupressus sempervirens;*
iii. une huile de fleur de *Chamaemelum nobile;*
iv. une huile de fleur d'*Helichrysum italicum;* et
v. une huile de Géranium bourbon
vi. de l'acétate d'α-tocophérol (vitamine E); puis
e) l'application sur la peau durant 20 à 30 minutes d'un masque dont le pH est de 6,3 et comprenant
i. du monostéarate de glycérol;
ii. de l'alcool cétylique;
iii. des triglycérides à chaînes moyennes;
iv. une composition comprenant de l'alcool cétéarylique et du sodium cétéaryl sulfate;
v. une composition comprenant de l'huile de graines de *Ricinus Communis,* de l'huile de ricin hydrogénée et de la cire de *Copernicia Cerifera*
vi. du beurre de karité;
vii. de l'extrait de concombre;
viii. du propylène glycol;
ix. de l'eau distillée;
x. du sorbate de potassium;
xi. un l'extrait d'*Alchemilla vulgaris,*
xii. une l'huile essentielle de *Citrus reticulata;* et
xiii. de l'acétate d'α-tocophérol (vitamine E); puis
f) le rinçage à l'eau tiède du masque appliqué dans l'étape e);
g) l'application sur la peau, à l'exception des yeux, des paupières et des lèvres, pendant 15 à 25 minutes, d'un masque à l'argile consistant en une pâte comprenant de l'eau mélangée à une argile verte ayant un contenu en Silicium compris entre 60 et 75% en poids et comprenant également du fer, du calcium, du manganèse, de l'azote et du potassium;
h) le retrait du masque à l'argile, puis
i) l'application sur la peau d'une crème comprenant
i. du monostéarate de glycérol;
ii. de l'alcool cétylique;
iii. des triglycérides saturés à chaînes moyennes;
iv. une composition comprenant de l'alcool cétéarylique et du sodium cétéaryl sulfate;
v. une composition comprenant de l'huile de graines de *Ricinus Communis,* de l'huile de ricin hydrogénée et de la cire de *Copernicia Cerifera;*
vi. du palmitate cétylique;
vii. du glycérol;
viii. du propylène glycol;
ix. de l'eau distillée;
x. du sorbate de potassium;
xi. un extrait liquide d'*Alchemilla vulgaris,*
xii. de l'alcool benzylique;
xiii. une huile essentielle de *Citrus reticulata;*
xiv. de l'acétate d'α-tocophérol (vitamine E); et
xv. de l'oxyde de zinc.

4. Procédé selon la revendication 3, où le peeling est appliqué dans l'étape b) à la main par des mouvements circulaire durant une période de 2 à 3 minutes.

5. Procédé selon la revendication 3 ou 4, où la peau est la peau du visage et/ou du décolleté.

6. Procédé selon l'une quelconque des revendications 3 à 5,
où une lotion énergisante est appliquée entre l'étape a) et l'étape b); et/ou où un sérum régénérant est appliqué sur la peau entre l'étape h) et l'étape i).

7. Un kit selon la revendication 2 ou un procédé selon la revendication 6,
où la lotion énergisante comprend :
i. de l'eau de rose concentrée, de préférence à une concentration de 1à 2% en poids, de préférence à une concentration de 1,2% en poids;
ii. de l'eau de fleurs d'oranger concentrée, de préférence à une concentration de 2,5 à 7,5% en poids, de préférence à 5,1% en poids;
iii. de l'eau distillée, de préférence à une concentration de 82 à 96% en poids, de préférence à une concentration de 88% en poids;
iv. de l'extrait de pépins de pamplemousse, de préférence à une concentration de 1,5 à 3,2% en poids, de préférence à une concentration de 2,1% en poids;
v. du sorbate de potassium, de préférence à une concentration de 1,5 à 2,5% en poids, de préférence à une concentration de 1,9% en poids; et
vi. du benzoate de sodium, de préférence à une concentration de 1,5 à 2,5% en poids, de préférence à une concentration de 1,7% en poids; et/ou
où le sérum régénérant comprend :
i. de l'acide hyaluronique, de préférence à une concentration de 0,1 à 0,3 % en poids, de préférence à une concentration de 0,25 % en poids;
ii. de l'extrait de concombre, de préférence dans le propylène glycol, de préférence à une concentration de 2,5 à 7,5 % en poids, de préférence à une concentration de 6 % en poids;
iii. de l'hydrolat de bleuet, de préférence à une concentration de 3 à 9 % en poids, de préférence à une concentration de 7 % en poids;
iv. du sorbate de potassium, de préférence à une concentration de 0,25 à 0,75 % en poids, de préférence à une concentration de 4 % en poids;
v. de l'huile essentielle de *Chamaemelum nobile* (camomille romaine), de préférence à une concentration de 0,05 à 0,2 % en poids, de préférence à une concentration de 0.2 % en poids;
vi. du xanthane, de préférence à une concentration de 0,25 à 0,75 % en poids, de préférence à une concentration de 0,15 % en poids; et
vii. de l'eau distillée, de préférence à une concentration de 83,7 à 87,5 % en poids, de préférence à une concentration de 86 % en poids.

8. Un kit selon l'une quelconque des revendications 1, 2 ou 7 ou un procédé selon l'une quelconque des revendications 3 à 7,
où le lait démaquillant et nourrissant comprend
i. de 66 à 85% en poids, de préférence 79.96% en poids, d'eau ;
ii. de 8 à 15% en poids, de préférence 8,4% en poids, de triglycérides saturés à chaîne moyenne ;
iii. de 6 à 10% en poids, de préférence 7% en poids, de monostéarate de glycérol ;
iv. de 1 à 1,5 % en poids, de préférence 1,25% en poids, de propylène glycol ;
v. de 1,2 à 1,8% en poids, de préférence 1,4% en poids, d'alcool cétylique ;
vi. de 0,1 à 0,5% en poids, de préférence 0,3% en poids, de diméticone ;
vii. de 0,15 à 0,4% en poids, de préférence 0,25% en poids, d'huile essentielle de mandarine verte ;
viii. de 0,02 à 0.08% en poids, de préférence 0,05% en poids, d'acétate d'α-tocophérol (vitamine E) ;
ix. de 0,15 à 0,45% en poids, de préférence 0,3% en poids, de carbopol ;
x. de 0,02 à 0,1% en poids, de préférence 0,03% en poids, de sorbate de potassium ;
xi. de 0,05 à 0,1% en poids, de préférence 0,06% en poids, d'EDTA ; et
xii. de 0,5 à 1,3% en poids, de préférence 1% en poids, d'hydroxyde de sodium ;
où la composition pour le traitement des capillaires comprend
i. de 78 à 92,8 % en poids, de préférence 87 % en poids, d'une huile végétale de *Rosa moschata* (rose musquée) ;
ii. de 4 à 12 % en poids, de préférence 7,74 % en poids, d'une huile de *Cupressus sempervirens* (cyprès) ;
iii. de 2,5 à 7 % en poids, de préférence 4,05 % en poids, d'une huile de fleur de *Chamaemelum nobile* (camomille romaine) ;
iv. de 0,4 à 1,2 % en poids, de préférence 0,6 % en poids, d'une huile de fleur d'*Helichrysum italicum* (helichryse italienne) ;
v. de 0,3 à 1 % en poids, de préférence 0,6 % en poids, d'une huile de Géranium bourbon ; et
vi. de 0,004 à 0,02, de préférence 0,01 % en poids, d'acétate d'α-tocophérol (vitamine E);
où le peeling comprend
i. de 2 à 8 % en poids, de préférence 6,4 % en poids, de monostéarate de glycérol ;
ii. de 3,5 à 7 % en poids, de préférence 5,66 % en poids, d'alcool cétylique ;
iii. de 4 à 8 % en poids, de préférence 6,02 % en poids, de triglycérides saturés à chaines moyennes ;
une concentration totale de 1,5 à 4,5 % en poids, de préférence une concentration totale de 3 % en poids, d'une composition comprenant de l'alcool cétéarylique et du sodium cétéaryl sulfate ;
v. de 5 à 12,5 % en poids, de préférence 10 % en poids, de propylène glycol ;
vi. de 50 à 80 % en poids, de préférence 61,5 % en poids, d'eau ;
vii. de 0,07 à 0,5 % en poids, de préférence 0,25 % en poids, de sorbate de potassium ;
viii. du xanthane, de préférence à une concentration;
ix. de 0,05 à 0,35 % en poids, de préférence 0,17 % en poids, d'acide salicylique ;
x. de 1 à 6 % en poids, de préférence 3 % en poids, d'un agent exfoliant contenant du polyéthylène et du stéarate de zinc ;
xi. de 0,02 à 0,4 % en poids, de préférence 0,2 % en poids, d'un huile essentielle de *Cymbopogon martini* ; et
xii. de 0,05 à 0,3 % en poids, de préférence 0,2 % en poids, d'une huile essentielle de menthe ;
où le masque dont le pH est de 6,3 comprend:
i. de 2 à 6 % en poids, de préférence 4 % en poids, de monostéarate de glycérol;
ii. de 3 à 8 % en poids, de préférence 6 % en poids, d'alcool cétylique;
iii. des triglycérides à chaînes moyennes;
iv. de 4 à 9 % en poids, de préférence 7,5 % en poids, d'une composition comprenant de l'alcool cétéarylique et du sodium cétéaryl sulfate;
v. de 10 à 25 % en poids, de préférence 15 % en poids, d'une composition comprenant de l'huile de graines de *Ricinus Communis,* de l'huile de ricin hydrogénée et de la cire de *Copernicia Cerifera*
vi. de 1,5 à 4 % en poids, de préférence 2,5 % en poids, de beurre de karité;
vii. de 5 à 10 % en poids, de préférence 8 % en poids, d'extrait de concombre;
viii. de 2,5 à 7,5 % en poids, de préférence 5 % en poids, de propylène glycol;
ix. de 20 à 64 % en poids, de préférence 34,65 % en poids, d'eau distillée;
x. de 0,005 à 0,3 % en poids, de préférence 0,15 % en poids, de sorbate de potassium;
xi. de 5 à 10 % en poids, de préférence 8 % en poids, d'extrait *d'Alchemilla vulgaris,*
xii. de 0,1 à 0,4 % en poids, de préférence 0,2 % en poids, d'une huile essentielle de *Citrus reticulata;* et
xiii. de 2,5 à 7,5 % en poids, de préférence 5 % en poids, d'acétate d'α-tocophérol (vitamine E); et/ou
où la crème comprend
i. de 2 à 5 % en poids, de 3,5 % en poids, de monostéarate de glycérol ;
ii. de 2 à 6 % en poids, de préférence 2,54 % en poids, d'alcool cétylique ;
iii. de 4 à 7 % en poids, de préférence 5 % en poids, de triglycérides saturés à chaînes moyennes ;
iv. une concentration totale de 0,5 à 2,5 % en poids, de préférence une concentration totale de 1 % en poids, d'une composition comprenant de l'alcool cétéarylique et du sodium cétéaryl sulfate ;
v. une concentration totale de 7 à 15 % en poids, de préférence une concentration totale de 10 % en poids, d'une composition comprenant de l'huile de graines de *Ricinus Communis,* de l'huile de ricin hydrogénée et de la cire de *Copernicia Cerifera ;*
vi. de 2 à 5 % en poids, de préférence 3 % en poids, de palmitate cétylique ;
vii. de 2 à 6 % en poids, de préférence 4 % en poids, de glycérol ;
viii. de 5 à 12 % en poids, de préférence 7 % en poids, de propylène glycol ;
ix. de 25 à 73 % en poids, de préférence 58 % en poids, dl'eau distillée ;
x. de 0,05 à 0,3 % en poids, de préférence 0,15 % en poids, de sorbate de potassium ;
xi. de 2 à 8 % en poids, de préférence 3,3 % en poids, d'un extrait liquide *d'Alchemilla vulgaris,*
xii. de 0,001à 0,1 % en poids, de préférence 0,01 % en poids, d'alcool benzylique ;
xiii. de 0,05 à 0,6 % en poids, de préférence 0,3 % en poids, d'une huile essentielle de *Citrus reticulata ;*
xiv. de 0,01 à 0,3 % en poids, de préférence 0,2 % en poids, d'acétate d'α-tocophérol (vitamine E) ; et
xv. de 1 à 4 % en poids, de préférence 2 % en poids, d'oxyde de zinc.

9. Utilisation d'un kit selon l'une quelconque des revendications 1 à 3 ou 8 pour le traitement cosmétique de la peau du visage et/ou du décolleté.

10. Utilisation selon la revendication 9, où le kit est utilisé pour l'amélioration de l'apparence de la peau, pour l'amélioration de l'hydratation de la peau, pour la réduction des lentigines solaires, taches de rousseur et taches de vieillissement (taches brunes), pour la réduction de la ptose de la peau, pour la réduction des rides, pour le remplissage des sillons naso-géniens, pour l'amélioration des émotions, pour l'amélioration de l'équilibre psycho-émotionnel, pour la réduction de l'anxiété et/ou pour la réduction de l'irritabilité d'un individu.
